**Europäisches Patentamt**

**European Patent Office**

**Office européen des brevets**

(19)

(11) Publication number : **0 309 089 B1**

# (12) EUROPEAN PATENT SPECIFICATION

(45) Date of publication of patent specification :
**12.02.92 Bulletin 92/07**

(51) Int. Cl.⁵ : **C07C 15/00,** C07C 2/00,
B01J 29/28

(21) Application number : **88307379.3**

(22) Date of filing : **10.08.88**

(54) Process for converting aliphatics to aromatics over a gallium-activated zeolite.

(30) Priority : **25.08.87 US 89194**

(43) Date of publication of application :
**29.03.89 Bulletin 89/13**

(45) Publication of the grant of the patent :
**12.02.92 Bulletin 92/07**

(84) Designated Contracting States :
**BE DE FR GB IT NL**

(56) References cited :
**EP-A- 0 216 491**
**EP-A- 0 252 705**
**US-A- 4 180 689**

(73) Proprietor : **MOBIL OIL CORPORATION**
**3225 Gallows Road**
**Fairfax, Virginia 22037-0001 (US)**

(72) Inventor : **Nemet-Mavrodin, Margaret Iosif**
**503 Garwood Drive**
**Cherry Hill, N.J. 08003 (US)**

(74) Representative : **Jones, Alan John et al**
**CARPMAELS & RANSFORD 43 Bloomsbury**
**Square**
**London, WC1A 2RA (GB)**

## Description

This invention relates to a process for the conversion of a feedstock containing aliphatic hydrocarbons to aromatics in the presence of a zeolite catalyst containing gallium.

Zeolites containing exchanged or impregnated gallium have been proposed in the past as catalysts for the production of aromatic hydrocarbons from aliphatic hydrocarbons by passing the aliphatic hydrocarbon over the catalyst at an elevated temperature in the liquid or vapor phase.

US-A-4,180,689 and 4,334,114 disclose processes for the production of aromatics by contacting a $C_3$-$C_{12}$ hydrocarbon feedstock with a catalyst in which gallium is supported on an aluminosilicate, e.g. ZSM-5. After addition of the gallium, the catalyst is activated at a temperature of between 400° and 650°. US-A-4,304,686 discloses the aromatization of aliphatic hydrocarbons utilizing as catalyst a zeolite having a silica/alumina molar ratio of 10:1 to 500:1 in which at least some of the cations have been exchanged for gallium ions. The zeolite is calcined at a temperature of at least 300°C, suitably between 300° and 800°C, before being treated to effect the gallium exchange. US-A-4,350,835 discloses a catalytic process for converting a feedstock comprising a high percentage of ethane to aromatics employing as a catalyst a zeolite, e.g., ZSM-5, with a silica/alumina ratio of at least 12 and having incorporated therein a minor amount of gallium. The zeolite is calcined at 540°C before the incorporation of the gallium. EP-A-50,021 discloses a process for producing aromatic hydrocarbons by contacting a feedstock containing at least 70% weight of $C_2$ hydrocarbons with a catalyst comprising an aluminosilicate, e.g., ZSM-5, with a silica/alumina molar ratio of at least 5:1, impregnated or exchanged with gallium. After incorporation of the gallium, the catalyst is activated at a temperature of between 400°C and 650°C. US-A-4,487,843 discloses the conversion of hydrocarbon feedstocks to aromatics using a catalyst subjected during its preparation to a steam treatment followed by loading with a Group IIIb metal, especially gallium. The catalyst may also be calcined under dry conditions at or about 550°C one or more times in the course of its preparation, but if the calcination stage during the preparation of a zeolite is carried out at or about 550°C under substantially dry conditions, the resulting catalysts are stated to have a high initial activity in hydrocarbon conversion reactions but also to produce coke at a high rate and therefore deactivate rapidly.

According to the present invention a process for producing aromatic compounds comprises contacting a $C_3$+ feed, at least 50% wt. of which consists of paraffins, olefins and naphthenes having up to 12 carbon atoms, under conversion conditions, including a temperature of 450 to 700°C, a pressure of 0.5 to 70 bar and a weight hourly space velocity of 0.05 to 50 hr$^{-1}$, of a severity to bring about at least 80% conversion of said paraffins, olefins and naphthenes, with a catalyst comprising a zeolite having a constraint index of 1 to 12 and a silica/alumina molar ratio of 25 to 1000, said zeolite being associated with exchanged or impregnated gallium and having been calcined at a temperature of at least 700°C after said association.

It has been found that the described catalyst has a higher selectivity to BTX aromatics than a similar catalyst which has not been exposed to such high temperatures after loading with gallium. Furthermore, a similar catalyst which is calcined at a similar temperature prior to loading with gallium is significantly less active than the catalyst of this invention.

The severity of process conditions refers to the tendency of the regime which they establish to cause reaction to proceed, and is thus increased by increase in temperature or pressure or by decrease in space velocity. It is thus the cumulative effect of the settings of the values of these parameters which determines severity. According to advantageous embodiments of the invention the process may be operated at a pressure of 1 to 70, preferably 1 to 35, more preferably 1 to 7 bar; a space velocity of 0.1 to 50, preferably 0.1 to 20, more preferably 0.5 to 5 hr$^{-1}$; and a temperature of preferably 500 to 650°C, more preferably 525 to 600°C. These conditions are desirably selected to establish a severity of operation which brings about at least 85% conversion of feed paraffins, olefins and naphthenes.

The zeolite on which gallium is loaded in producing the catalyst may be prepared by any of the methods known in the art, including variations of the original method for the production of this type of zeolite utilizing an "organic template" provided by the presence of organic cations. The zeolite may indeed be prepared without employing any organic cations, possibly utilizing instead seeds of the desired zeolite in the formulating mixture which seeds themselves may have been formed in the presence of organic ions or from other seeds formed in the presence of organic ions. Moreover, the zeolites contemplated in the process of this invention may be formed in the absence of any organic ions or seeds of the type described, utilizing instead as precursor a silica or aluminosilicate which is precipitated or crystallized from solution or homogeneous amorphous phase and having certain characteristics, as disclosed for example in EP-A-106,552.

The process of the present invention may be carried out using catalysts in which gallium is impregnated on the surface of the zeolite or is ion-exchanged with the cations of the zeolite using techniques of impregnation or ion-exchange which are well-known in the art. For example, the gallium may be impregnated on the surface of the zeolite by preparing a solution, e.g, an aqueous solution of a gallium compound such as gallium nitrate and adding to this solution a preshaped form of the desired zeolite such as 1/16 in. extrudates with alumina as a binder, or in the form of a fluid bed powder, and allowing the zeolite to be thoroughly contacted with the gallium solution. The contacted catalyst is then dried under vacuum at a moderate temperature (90-100°C). After calcination, the zeolite contains gallium impregnated on its surface in the form of gallium oxide.

The gallium in the catalyst composition is present as ions if the cations in the aluminosilicate support have been exchanged with gallium ions. In this case, the gallium ions are suitably provided as an aqueous solution of a gallium salt such as for instance gallium sulfate, nitrate, or chloride. Such catalysts may be produced by conventional ion exchange techniques and the catalysts so produced are subsequently dried. For example, an aqueous solution of gallium compound such as gallium sulfate may be placed in contact with the ammonium form of a preshaped form of the zeolite at ambient or elevated temperature, e.g., by refluxing. The exchanged zeolite is then separated by decantation followed by filtration, washed several times with deionized water and finally dried.

When the catalyst composition is prepared by using a compound of gallium-comprising metal which ionizes in aqueous solution, for example gallium nitrate, some of the gallium ions are generally exchanged with the cations in the zeolite even if the preparation was directed to impregnation.

Whichever method of catalyst preparation is used, the amount of gallium present in the total catalyst composition may vary, for example, between about 0.5 and 5 percent by weight, preferably between about 0.5 and 2.0 percent by weight. Elements other than gallium may also be present, such as any of various suitable metals in Groups I through VIII of the Periodic Table including by way of example zinc, platinum, rhenium, cobalt, titanium, tellurium, sodium, nickel, chromium, aluminum, copper, platinum, calcium and rare earth metals or other modifiers, such as phosphorus. In general, the amount of gallium added to the zeolite will be more than 50% by weight of the total added metal.

As stated, for purposes of this invention the gallium-loaded zeolite catalyst must be calcined at a temperature of at least about 700°C, before being used as a catalyst for the aromatization of aliphatic compounds. For example, the catalyst may be heated at a temperature of about 800 to 825°C for a period of about 1 to 1.5 hours prior to use.

The significance and manner of determination of Constraint Index are described in US-A-4,016,218. Constraint Index (CI) values for some typical materials are:

|  | _CI (at test temperature)_ |  |
|---|---|---|
| ZSM-4 | 0.5 | (316°C) |
| ZSM-5 | 6-8.3 | (371°C - 316°C) |
| ZSM-11 | 5-8.7 | (371°C - 316°C) |
| ZSM-12 | 2.3 | (316°C) |
| ZSM-20 | 0.5 | (371°C) |
| ZSM-22 | 7.3 | (427°C) |
| ZSM-23 | 9.1 | (427°C) |
| ZSM-34 | 50 | (371°C) |
| ZSM-35 | 4.5 | (454°C) |
| ZSM-38 | 2 | (510°C) |
| ZSM-48 | 3.5 | (538°C) |
| ZSM-50 | 2.1 | (427°C) |
| TMA Offretite | 3.7 | (316°C) |
| TEA Mordenite | 0.4 | (316°C) |
| Clinoptilolite | 3.4 | (510°C) |
| Mordenite | 0.5 | (316°C) |
| REY | 0.4 | (316°C) |
| Amorphous Silica-alumina | 0.6 | (538°C) |
| Dealuminised Y | 0.5 | (510°C) |
| Erionite | 38 | (316°C) |
| Zeolite Beta | 0.6-2.0 | (316°C-399°C) |

The nature of this parameter and of the technique by which it is determined admit the possibility that a given zeolite can be tested under somewhat different conditions and thereby exhibit different Constraint Indices. For the purposes of this specification a zeolite is considered to possess a Constraint Index of 1 to 12 if under conditions which fall within the testing prescriptions set forth in US-A-4,016,218 it manifests a Constraint Index within that range even though under different conditions within those prescriptions it manifests a Constraint Index outside that range.

The zeolites preferred for use according to the invention are ZSM-5, ZSM-11, ZSM-12 , ZSM-23, ZSM-35, ZSM-38 and ZSM-48, defined respectively by the x-ray diffraction data set forth in US-A-3,702,886, 3,709,979, 3,832,449, 4,076,842, 4,016,245, 4,046,859 and 4,350,835.

Natural zeolites may sometimes be converted to zeolite structures of the class herein identified by various activation procedures and other treatments such as base exchange, steaming, alumina extraction and calcination, alone or in combinations. Natural minerals which may be so treated include ferrierite, brewsterite, stilbite, dachiardite, epistilbite, heulandite and clinoptilolite.

In practising the invention it may be advantageous to incorporate the zeolite, prior to modification with gallium, into a matrix comprising another material resistant to the temperature and other conditions employed in the process. Such matrix material is useful as a binder and imparts greater resistance to the catalyst for the severe temperature, pressure and reactant feed stream velocity conditions encountered in many conversion processes.

Useful matrix materials included both synthetic and naturally occurring substances, as well as inorganic materials such as clay, silica and/or metal oxides. The latter may be either naturally occurring or in the form of gelatinous precipitates or gels including mixtures of silica and metal oxides. Naturally occurring clays which can be composited with the zeolite include those of the montmorillonite and kaolin families, which families include the sub-bentonites and kaolins commonly known as Dixie, McNamee-Georgia and Florida clays or others in which the main mineral constituent is halloysite, kaolinite, kickite, nacrite or anauxite. Such clays can be used in the raw state as originally mined or initially subjected to calcination, acid treatment or chemical modification.

In addition to the foregoing materials, the zeolites employed herein may be composited with a porous matrix material, such as alumina, silica-alumina, silica-magnesia, silica-zirconia, silica-thoria, silica-beryllia and silica-titania, as well as ternary compositions, such as silica-alumina-magnesia and silica-magnesia-zirconia. The matrix may be in the form of a cogel. The relative proportions of zeolite component and inorganic oxide gel matrix, on an anhydrous basis, may very widely, with the zeolite content ranging from between about 1 to about 99 percent by weight and more usually in the range of about 25 to about 65 percent by weight of the dry composite.

The feed stream to the process of this invention contains at least 50% by weight of at least one aliphatic hydrocarbon containing 3 to 12 carbon atoms. The hydrocarbon may be straight chain, open or cyclic and may be saturated or unsaturated. Some contemplated hydrocarbons are propane, propylene, n-butane, n-butenes, isobutane, isobutene, and straight- and branch-chain and cyclic pentanes, pentenes, hexanes, hexenes, heptanes, heptenes, octanes, octenes, nonanes, nonenes, decanes and decenes. In a preferred embodiment $C_5$-$C_8$ paraffins constitute 75% wt. of the feed.

The process of this invention is conducted so that a feed containing a high percentage, i.e., at least 50 wt.% of $C_3$-$C_{12}$ aliphatic hydrocarbons, is contacted with a catalyst of this invention in a reaction zone, such as, for example, a fixed bed of catalyst composition under effective conversion conditions. In a typical embodiment of the process in this invention, the feed stream is introduced into the reaction zone at a temperature within the range of about 450°C to 700°C a pressure within the range of about one atmosphere to 70 bar (1000psig) and a WHSV of about 0.1 to 50.

The effluent from the reaction zone is separated and distilled to remove the desired aromatic product and the remainder is recycled for further reaction.

The process of this invention may be carried out with the catalyst in the form of a fixed, moving, or fluidized bed.

The following examples further illustrate the invention.

Comparative Example A

Ten grams of a zeolite composition comprising 25 wt.% of a ZSM-5 zeolite having a silica/alumina molar ratio of 55:1 and prepared as described in EP-A-130,809, and 75 wt.% of kaolin as a binder, were calcined in air to 500°C for 5 hours to remove any residual organic matter. It was then exchanged with 50 ml. of 0.5 M aqueous ammonia solution at 90°C for 4 hours, washed with deionized water, and again exchanged with the same ammonia solution overnight at room temperature. After this, the zeolite was again washed with deionized water, dried under vacuum, and exchanged with the 30 ml of 0.35 M aqueous $Ga(NO_3)_3$ solution at reflux for 3 hours. After washing the zeolite three times with 50 ml. of deionized water, it was exchanged again with the 0.35 M $Ga(NO_3)_3$ solution as described, and again washed with deionized water as described. The gallium-exchanged zeolite was dried under 74 mm. Hg vacuum (0.1 bar) at 90°C overnight and calcined in air at 500°C in a muffle furnace for 5 hours to obtain a gallium-loaded, ZSM-5 zeolite catalyst containing 1.94% of gallium based on the weight of the catalyst.

10 grams of the catalyst was contacted at 550°C and atmospheric pressure in a laboratory microreactor with a highly paraffinic feedstock which had been vaporized and heated to reactor temperature. The feedstock had the composition indicated in Table I.

## Table I

| Component | Wt.% |
|---|---|
| $C_4$ Aliphatics | 0.1 |
| $C_5$ Aliphatics | 4.7 |
| $C_6$ Paraffins | 51.4 |
| $C_6$ Olefins & Naphthenes | 3.4 |
| $C_7$ Paraffins | 32.3 |
| $C_7$ Olefins & Naphthenes | 0.3 |
| $C_8$ Aliphatics | 1.2 |
| $C_9+$ Aliphatics | 2.3 |
| Benzene | 0.2 |
| Toluene | 4.0 |
| Xylenes | 0.1 |

The feed stream was passed over the catalyst at a WHSV (including binder) of 0.63 which resulted in a reaction time of 17.7 seconds. After various times on stream, the product was analyzed. Hydrogen and light gases ($C_1$-$C_3$) were analyzed by refinery gas analysis GC, and complete hydrocarbon analysis was obtained on a CP Sil 5CB capillary column. The results are shown in Table II, where "PON conversion" is the percent conversion of the total of the paraffins, olefins and naphthenes in the feed to any products, the selectivity of any of the indicated products is the percentage by weight of that product based on the total products resulting from the PON conversion, and "BTX" is the total of the $C_6$-$C_8$ aromatics produced, including benzene, toluene, xylenes and ethyl benzene. The other listed product components are self-explanatory.

## Table II

| Time on Stream, hrs | 0.9 | 2.3 | 3.8 |
|---|---|---|---|
| PON Conversion, % | 95.2 | 93.0 | 89.7 |
| Selectivity, wt.% | | | |
| BTX | 48.2 | 49.1 | 47.1 |
| $C_2$-$C_4$ Olefins | 5.2 | 6.3 | 8.9 |
| $C_9+$ Aromatics | 12.3 | 11.5 | 10.5 |
| $C_1 + C_2$ Paraffins | 16.9 | 15.7 | 15.4 |
| Propane | 14.0 | 14.2 | 15.1 |

EXAMPLE 1

The procedure of Comparative Example A was followed except that the described gallium-loaded ZSM-5 zeolite catalyst was calcined in flowing air at 800°C for one hour before use. The gallium content was unchanged. The results are shown in Table III.

## Table III

| Time on Stream, hrs | 1.6 | 3.1 | 4.6 | 6.1 | 7.7 |
|---|---|---|---|---|---|
| PON Conversion, % | 90.9 | 88.2 | 86.7 | 85.4 | 84.3 |
| Selectivity, wt.% | | | | | |
| BTX | 55.2 | 53.3 | 52.5 | 51.6 | 50.9 |
| $C_2$-$C_4$ Olefins | 7.2 | 9.7 | 11.1 | 12.6 | 13.6 |
| $C_9$+ Aromatics | 8.6 | 8.3 | 8.4 | 8.4 | 8.5 |
| $C_1$ + $C_2$ Paraffins | 12.6 | 12.5 | 12.1 | 11.9 | 11.7 |
| Propane | 13.0 | 12.8 | 12.4 | 12.2 | 12.1 |

Example 2

The procedure of Comparative Example A was followed except that the ZSM-5 zeolite was exchanged only once with $Ga(NO_3)_3$ solution as described, to prepare a gallium-loaded ZSM-5 zeolite, containing 1.1% of gallium based on the weight of the catalyst, which was calcined in flowing air at 800°C for one hour, and the feed stream was passed over the catalyst bed at a WHSV of 0.66 for a reaction time of 17.0 seconds. The results are shown in Table IV:

## Table IV

| Time on Stream, hrs | 0.4 | 1.9 | 3.4 | 4.9 | 6.4 |
|---|---|---|---|---|---|
| PON Conversion, % | 96.1 | 93.6 | 91.9 | 91.6 | 90.8 |
| Selectivity, wt.% | | | | | |
| BTX | 54.9 | 52.9 | 52.7 | 52.7 | 52.4 |
| $C_2$-$C_4$ Olefins | 5.2 | 7.6 | 9.4 | 9.7 | 10.6 |
| $C_9$+ Aromatics | 7.9 | 8.7 | 6.3 | 6.5 | 6.8 |
| $C_1$ + $C_2$ Paraffins | 15.3 | 14.5 | 14.7 | 14.5 | 14.0 |
| Propane 13.0 | 13.2 | 12.9 | 13.4 | 13.2 | 12.9 |

Comparison of the results of Comparative Example A, wherein the gallium-loaded ZSM-5 was calcined at 500°C, with those of Examples 1 and 2 wherein the gallium-loaded zeolite was calcined at 800°C, indicates that calcining the gallium-loaded zeolite at a temperature of at least 700°C, e.g., 800°C, results in higher selectivities to BTX aromatics than are obtained when calcination of the gallium-loaded zeolite at a temperature of at least 700°C is not carried out.

Selectivities similar to those of Examples 1 and 2 were obtained when the gallium was loaded on the ZSM-5 zeolite by impregnation rather than ion exchange.

Comparative Example B

The procedure of Comparative Example A was followed except that the ZSM-5 zeolite after being calcined at 500°C to remove residual organics and before being exchanged with gallium, was calcined in flowing air at 850°C for one hour. The gallium-loaded catalyst contained 0.92% gallium based on the weight of the catalyst. As in Comparative Example A, there was no calcining of the catalyst at 800°C after the exchange with gallium. In addition, in order to obtain equivalent conversions, the feedstock was passed over the catalyst at the much lower WHSV of 0.14, resulting in a reaction time of 78.8 seconds. The results are shown in Table V:

## Table V

| Time on Stream, hrs | 1.2 | 2.7 | 4.2 | 5.7 | 7.2 | 9.1 |
|---|---|---|---|---|---|---|
| PON Conversion, % | 96.2 | 94.3 | 93.3 | 91.9 | 91.9 | 89.5 |
| Selectivity, wt.% | | | | | | |
| BTX | 55.7 | 51.4 | 51.8 | 49.8 | 51.6 | 45.7 |
| $C_2$-$C_4$ Olefins | 1.3 | 2.1 | 2.4 | 3.1 | 3.2 | 4.5 |
| $C_9$+ Aromatics | 8.1 | 10.8 | 11.0 | 13.2 | 9.8 | 9.4 |
| $C_1$ + $C_2$ Paraffins | 15.6 | 15.7 | 14.8 | 14.2 | 15.0 | 16.8 |
| Propane 13.0 | 14.4 | 14.1 | 13.4 | 12.6 | 13.0 | 14.4 |

Comparative Example C

The procedure of Comparative Example B was followed except that the feed rate was 0.31 WHSV equivalent to a reaction time of 36.7 seconds. The results are shown in Table VI:

## Table VI

| Time on Stream, hrs | 2.4 | 3.9 | 5.4 | 6.9 | 8.6 |
|---|---|---|---|---|---|
| PON Conversion, % | 85.9 | 84.1 | 81.4 | 80.0 | 78.6 |
| Selectivity, wt.% | | | | | |
| BTX | 52.6 | 52.1 | 51.1 | 50.6 | 49.9 |
| $C_2$-$C_4$ Olefins | 9.0 | 10.5 | 12.1 | 13.3 | 14.2 |
| $C_9$+ Aromatics | 8.1 | 8.4 | 8.3 | 8.3 | 8.0 |
| $C_1$ + $C_2$ Paraffins | 14.2 | 13.7 | 13.5 | 13.3 | 13.3 |
| Propane | 13.1 | 12.3 | 11.9 | 11.7 | 11.7 |

A comparison of the results of Examples 1 and 2 with those of Comparative Examples B and C shows that a catalyst in accordance with the invention has substantially greater activity for the aromatization of feedstocks having a high proportion of $C_3$- $C_{12}$ aliphatics, with equivalent or better BTX selectivity, than a gallium-loaded catalyst prepared by calcining a similar ZSM-5 zeolite at a temperature higher than 700°C before rather than after loading the zeolite with gallium. That the latter catalyst has substantially lower activity than the catalyst of the invention is confirmed by comparing the results of Comparative Examples B and C, where the feed rate was raised from 0.14 WHSV in Comparative Example B to 0.31 WHSV in Comparative Example C (which was still less than one half that of Examples 1 and 2), the PON conversion activity of the catalyst dropped considerably without any significant increase in BTX selectivity.

**Claims**

1. A process for producing aromatic compounds which comprises contacting a $C_3$+ feed, at least 50% wt. of which consists of paraffins, olefins and naphthenes having up to 12 carbon atoms, under conversion conditions, including a temperature of 450 to 700°C, a pressure of 0.5 to 70 bar and a weight hourly space velocity of 0.05 to 50 $hr^{-1}$, of a severity to bring about at least 80% conversion of said paraffins, olefins and naphthenes, with a catalyst comprising a zeolite having a constraint index of 1 to 12 and a silica/alumina molar ratio of 25 to 1000, said zeolite being associated with exchanged or impregnated gallium and having been calcined at a temperature of at least 700°C after said association.

2. A process according to claim 1 wherein the pressure is 1 to 70 bar and the space velocity is 0.1 to 50 hr$^{-1}$.

3. A process according to claim 1 or claim 2 wherein the temperature is 500 to 650°C, the pressure is 1 to 35 bar and the space velocity is 0.1 to 20 hr$^{-1}$.

4. A process according to any preceding claim wherein the temperature is 525 to 600°C, the pressure is 1 to 7 bar and the space velocity is 0.5 to 5 hr$^{-1}$.

5. A process according to any preceding claim wherein the severity of said conditions brings about at least 85% conversion of said paraffins, olefins and naphthenes.

6. A process according to any preceding claim wherein said catalyst is in the form of a fixed bed.

7. A process according to any preceding claim wherein at least 75% wt. of said $C_3$+ feed consists of $C_5$-$C_8$ paraffins.

8. A process according to any preceding claim wherein gallium constitutes 0.5 to 5% wt. of the exchanged or impregnated zeolite.

9. A process according to any preceding claim wherein the temperature of calcination of the zeolite is at least 775°C.

10. A process according to claim 9 wherein said calcination is carried out at 800 to 825°C for from 1 to 1.5 hours.

11. A process according to any preceding claim wherein the zeolite is ZSM-5, -11, -12, -23, -35, -38 or -48.

12. A process according to any preceding claim wherein the zeolite constitutes 1 to 99% wt. of a composite with an inorganic oxide matrix.

13. A process according to claim 12 wherein the zeolite constitutes 25 to 65% wt. of the composite.


**Patentansprüche**

1. Verfahren zur Herstellung aromatischer Verbindungen, das den Kontakt einer $C_3$+ -Zufuhr, wobei mindestens 50 Gew.-% davon aus Paraffinen, Olefinen und Naphthenen mit bis zu 12 Kohlenstoffatomen bestehen, bei Umwandlungsbedingungen, die eine Temperatur von 450 bis 700°C, einen Druck von 0,5 bis 70 bar und eine stündliche Gewichts-Raum-Geschwindigkeit von 0,05 bis 50 h$^{-1}$ umfassen, bei einer Severity um eine mindestens 80%-ige Umwandlung der Paraffine, Olefine und Naphthene zu bewirken, mit einem Katalysator umfaßt, der einen Zeolith mit einem Zwangsindex von 1 bis 12 und einem Siliciumdioxid/Aluminiumoxid-Molverhältnis von 25 bis 1000 umfaßt, wobei der Zeolith mit ausgetauschtem oder imprägniertem Gallium verbunden ist und nach dieser Verbindung bei einer Temperatur von mindestens 700°C kalziniert wurde.

2. Verfahren nach Anspruch 1, worin der Druck 1 bis 70 bar und die Raum-Geschwindigkeit 0,1 bis 50 h$^{-1}$ betragen.

3. Verfahren nach Anspruch 1 oder 2, worin die Temperatur 500 bis 650°C, der Druck 1 bis 35 bar und die Raum-Geschwindigkeit 0,1 bis 20 h$^{-1}$ betragen.

4. Verfahren nach einem der vorstehenden Ansprüche, worin die Temperatur 525 bis 600 °C, der Druck 1 bis 7 bar und die Raum-Geschwindigkeit 0,5 bis 5 h$^{-1}$ betragen.

5. Verfahren nach einem der vorstehenden Ansprüche, worin die Severity dieser Bedingungen eine mindestens 85%ige Umwandlung der Paraffine, Olefine und Naphthene bewirkt.

6. Verfahren nach einem der vorstehenden Ansprüche, worin der Katalysator in Form eines Festbetts vorliegt.

7. Verfahren nach einem der vorstehenden Ansprüche, worin mindestens 75 Gew.-% der $C_3$+-Zufuhr aus $C_5$-$C_8$-Paraffinen bestehen.

8. Verfahren nach einem der vorstehenden Ansprüche, worin das Gallium 0,5 bis 5 Gew.-% des ausgetauschten oder imprägnierten Zeoliths bildet.

9. Verfahren nach einem der vorstehenden Ansprüche, worin die Temperatur der Kalzinierung des Zeoliths mindestens 775°C beträgt.

10. Verfahren nach Anspruch 9, worin die Kalzinierung 1 bis 1,5 Stunden lang bei 800 bis 825°C durchgeführt wird.

11. Verfahren nach einem der vorstehenden Ansprüche, worin der Zeolith ZSM-5, -11, -12, -23, -35, -38 oder -48 ist.

12. Verfahren nach einem der vorstehenden Ansprüche, worin der Zeolith 1 bis 99 Gew.-% eines Verbundmaterials mit einer anorganischen Oxidmatrix bildet.

13. Verfahren nach Anspruch 12, worin der Zeolith 25 bis 65 Gew.-% des Verbundmaterials bildet.

**Revendications**

1. Un procédé de production de composés aromatiques qui comprend la mise en contact d'une charge en C$_3$+, 50% en poids au moins de celle-ci consistant en paraffines, oléfines et naphtènes ayant jusqu'à 12 atomes de carbone, dans des conditions de conversion, incluant une température de 450 à 700°C, une pression de 0,5 à 70 bars et une vitesse spatiale horaire pondérale de 0,05 à 50 h$^{-1}$, d'une sévérité telle qu'environ au moins 80% desdites paraffines, oléfines et naphtènes sont convertis, en présence d'un catalyseur comprenant une zéolite ayant un indice de contrainte de 1 à 12 et un rapport molaire silice/alumine de 25 à 1 000, ladite zéolite étant associée à du gallium provenant d'un traitement d'échange ou d'imprégnation et ayant été calcinée à une température d'au moins 760°C après ladite association.

2. Un procédé selon la revendication 1, dans lequel la pression est de 1 à 70 bars et la vitesse spatiale de 0,1 à 50 h$^{-1}$.

3. Un procédé selon la revendication 1 ou 2, dans lequel la température est de 500 à 650°C, la pression de 1 à 35 bars et la vitesse spatiale de 0,1 à 20 h$^{-1}$.

4. Un procédé selon l'une quelconque des revendications précédentes, dans lequel la température est de 525 à 600°C, la pression de 1 à 7 bars et la vitesse spatiale de 0,5 à 5 h$^{-1}$.

5. Un procédé selon l'une quelconque des revendications précédentes, dans lequel la sévérité desdites conditions est telle qu'au moins 85% desdites paraffines, oléfines et naphtènes sont convertis.

6. Un procédé selon l'une quelconque des revendications précédentes, dans lequel ledit catalyseur est sous la forme d'un lit fixe.

7. Un procédé selon l'une quelconque des revendications précédentes, dans lequel au moins 75% de ladite charge en C$_3$+ consistent en des paraffines en C$_5$ à C$_8$.

8. Un procédé selon l'une quelconque des revendications précédentes, dans lequel le gallium constitue de 0,5 à 5% en poids de la zéolite ayant subi un traitement d'échange ou d'imprégnation.

9. Un procédé selon l'une quelconque des revendications précédentes, dans lequel la température de calcination de la zéolite est d'au moins 775°C.

10. Un procédé selon la revendication 9, dans lequel ladite calcination est mise en oeuvre à une température de 800 à 825°C pendant 1 à 1,5 heures.

11. Un procédé selon l'une quelconque des revendications précédentes, dans lequel la zéolite est de la ZSM-5, -11, -12, -23, -35, -38 ou -58.

12. Un procédé selon l'une quelconque des revendications précédentes, dans lequel la zéolite constitue de 1 à 99% en poids d'un composite avec une matrice à base d'oxyde inorganique.

13. Un procédé selon la revendication 12, dans lequel la zéolite constitue de 25 à 65% en poids du composite.